# EUROPEAN PATENT APPLICATION

(11) **EP 0 580 236 A2**
(43) Date of publication of application: **26.01.1994**
(21) Application number: 93202103.3
(22) Date of filing: 19.07.1993
(51) Int. Cl.: A23K 3/03, C12N 1/20

(54) **Silage inoculant**

(30) Priority: 24.07.1992 EP 92202272
(71) Applicant: DUPHAR INTERNATIONAL RESEARCH B.V, NL-1380 AC Weesp (NL)
(72) Inventor: ten Brink, Bart, NL-1380 AC Weesp (NL); Spoelstra, Sierk F., NL-1380 AC Weesp (NL); van Dasler, Hans K., NL-1380 AC Weesp (NL)
(74) Representative: Breepoel, Peter M.

(57) **Abstract**

The present invention is concerned with a method for the production of silage by microbial conversion of silage crop during an aerobic phase and a subsequent anaerobic phase, wherein the anaerobic phase takes place in the presence of lactic acid bacteria, preferably Lactobacillus plantarum bacteria which produce a Clostridium-inhibiting factor, in particular a proteinaceous Clostridium-inhibiting factor.
The invention is also concerned with novel Lactobacillus plantarum species which produce a Clostridium-inhibiting factor, in particular a proteinaceous Clostridium-inhibiting factor.

## Description

The present invention is concerned with a method for the production of silage by microbial conversion of silage crop during a first aerobic and a subsequent anaerobic phase.

The quality of silage is subject to wide variation. Fluctuations in weather conditions are generally considered to be the main factor causing such variations. Improvement of the quality of silage is often attained by addition of bacterial inoculants and other silage additives. However, these additions turn out to have a minor impact on silage quality under moderate or bad conditions.

It is an object of the present invention to provide a silage additive which results in silage of good quality under various weather conditions.

When silage crop is cut off from the air, a number of microbial processes take place. During a first aerobic phase the obligate and facultative aerobic bacteria remain active. These bacteria convert hexose and oxygen into carbon dioxide and water. As soon as the oxygen is depleted the obligate aerobic bacteria lose their activity. As long as the pH stays above 5 the entero bacteria and the yeast convert hexose into fatty acids, ethanol and carbon dioxide. However, the lactic acid bacteria present will produce lactic acid which will gradually decrease the pH value below 4.

At pH4 and below bacterial activity will cease, and hence the silage will remain well preserved.

However, in many cases the pH lowering occurs too slowly. Many causes may underly this phenomenon. If the weather prevents fast drying of the silage crop the respiration of the plants will continue for too long. The sugars present will then be digested and too little substrate will remain for sufficient bacterial activity. Hence the pH lowering will be insufficient. Under these conditions of a slow pH lowering clostridia (i.e. Clostridium tyrobutyricum) start to convert part of the available substrate into butyric acid. Furthermore, these bacteria are able to convert the lactic acid previously produced into butyric acid. By this process two molecules of lactic acid are converted into one molecule of the weaker butyric acid, thereby leading to a rise of the pH. At this higher pH also the proteolytic clostridia remain active. These proteolytic clostridia convert protein into ammonia, which also contributes to a pH rise.

As a result, the quality of the silage in terms of nutritional value will be much lower (less dry material, less proteins).

Furthermore, the occurence of clostridia in silage will cause the presence of Clostridium spores in raw milk from cows fed with this silage. This Clostridium contamination will have a negative influence on the quality of milk products, in particular on the quality of cheese.

It has been found now that the nutritional value of silage produced under unfavorable weather conditions can be improved by inoculation of the silage crop with lactic acid bacteria which are able to produce a Clostridium-inhibiting factor.

Advantageously, use can be made of Lactobacillus plantarum bacteria which are able to produce a high molecular weight Clostridium-inhibiting factor, more in particular a proteinaceous factor.

In general, such a factor is characterized in that its Clostridium-inhibiting activity can be abolished by trypsine and by heat treatment. The factor is unable to pass a ultrafiltration membrane having a cut-off value of 30 kD.

Examples of lactobacilli useful according to the present invention are the Lactobacillus plantarum strains G147 and F123, samples of which have been deposited at the Centraalbureau voor Schimmelcultures at Baarn, the Netherlands under the deposit numbers CBS 343.92 and CBS 342.92, respectively.

Suitable silage crops are grass, clover and alfalfa. These crops are stored in such a way that exposure to air is prevented, for instance in a silo or under plastic. When introduction of air is prevented, fermentation will occur. In order to enhance the rate of fermentation or to direct the type of fermentation, silage additives can be aded. Examples of silage additives are: sugars, enzymes, acids, salts and inoculants.

Silage inoculants for use according to the present invention are Lactobacillus plantarum cells with Clostridium-inhibiting activity, formulated as a dry or concentrated liquid product. This product is generally suspended in water and sprayed over the crops before and during ensiling. The culture can be applied in 10⁴ - 10⁸ colony forming units (cfu's) per gram of crop, preferably 10⁶ cfu's/g of crop.

### Example 1

### Propagation of Lactobacillus

Lactobacilli were isolated by plating appropriate dilutions of starting material on MRS agar plates. The composition of the RMS medium is given in Table 1 (De Man, J.C. Rogers, M. and Sharpe, M.E. (1960) J. Appl. Bacteriology 23, 130-135).

**Table 1**

| **Component** | **Amount** |
|---|---|
| Peptone | 10.0 g/l |
| "Lab-lemco" powder | 8.0 g/l |
| Yeast extract | 4.0 g/l |
| Glucose | 20.0 g/l |
| Tween 80 | 1.0 ml/l |
| K₂HPO₄ | 2.0 g/l |
| Sodium acetate.3H₂O | 5.0 g/l |
| Tri-ammonium citrate | 2.0 g/l |
| Magnesium sulphate.7H₂O | 0.2 g/l |
| Manganese sulphate.4H₂O | 0.05 g/l |

After 48 hours incubation under anaerobic conditions at 30 °C, isolated colonies were selected. The selected colonies were propagated in MRS broth supplemented with glucose (2% w/v) and calcium carbonate (6% w/v) for 48 hours at 30 °C under anaerobic conditions.

### Characterization of Clostridium-inhibiting factor

The Clostridium-inhibiting factors produced by the Lactobaccilus strains G 147 and F 123 were further characterized with regard to heat stability, trypsin sensitivity and molecular dimensions.

### Heat stability

Cell free supernatants of the respective Lactobacillus strains were incubated during a certain period at a certain temperature, and the remaining inhibiting activity was determined.

**Table 2**

| **Treatment** | **Inhibition zone (in mm) against Clostridium** | |
|---|---|---|
| | F123 | G147 |
| controle | 14 | 14 |
| 5 min 75°C | 14 | 14 |
| 10 min 75°C | 14 | 14 |
| 15 min 75°C | 14 | 14 |
| 5 min 100°C | 10 | 13 |
| 10 min 100°C | 8 | 12 |
| 15 min 100°C | 6 | 11 |
| 5 min 121°C | 8 | 9 |

The results (summarized in Table 2) show that the inhibitors investigated are resistent to pasteurization. However, they are inactivated by cooking and by sterilization.
The inhibitor produced by strain F123 seems to be slightly more heat-sensitive than the one produced by strain G147.

### Trypsin sensitivity

Trypsin (1 mg) was added to 1 ml each of the cell free supernatant of strains F123 and G147.
Subsequently, at pH 8, the supernatants were incubated during 1 hour at 37°C.
Thereafter the pH was lowered to 5.8 and the remaining clostridium inhibiting activity was determined.
In all of the cases tested the inhibiting activity had completely disappeared.

### Molecular dimensions

Cell free supernatants of strains F123 and G147 were subjected to ultrafiltration in a Centricon 30 filtersystem (Amicon). The two compartments of this filter system were separated by a filter with a cut-off of 30 kD.
Portions of 5 ml of each of the supernatants were transferred to the upper compartment of the filter system, and the system was subjected to a centrifugal force (10000 g) during 10 minutes and the inhibiting activities of the contents of both compartments [permeate (lower compartment); retentate (upper compartment)] were tested. The results are summarized in Table 3.

**Table 3**

| **Material** | **Inhibiting zone (in mm) against Clostridium sporogenes caused by strain:** | |
|---|---|---|
| | F123 | G147 |
| Starting material | 14 | 14 |
| permeate (<30 kD) | 6 | 6 |
| retentate (>30 kD) | 19 | 18 |

### Example 2

### Acidification of Grass

The grass was mowed and pre-dried at the field until the dry mass content was 30%. Subsequently the grass was cut up into pieces of about 2 cm.
The shredded grass was inoculated with 0, 10⁵, 10⁶ colony-forming units/gram of grass (cfu/g) of strain G147. Inoculation was carried out by spraying the bacteria suspension onto the grass (25 ml suspension/kg of grass) using a plant spray during mixing of the grass in a concrete mixer. For each treatment eight 1l bottles were filled with 400 g of grass each, and these bottles were stored at room temperature.

During the experiments for each treatment two bottles were opened at 0, 24, 48 and 72 hours of incubation time.
An aqueous extract (30 g of grass + 270 g of demineralized water, 5 minutes in Stomacher) was made from each bottle, and the pH was measured.

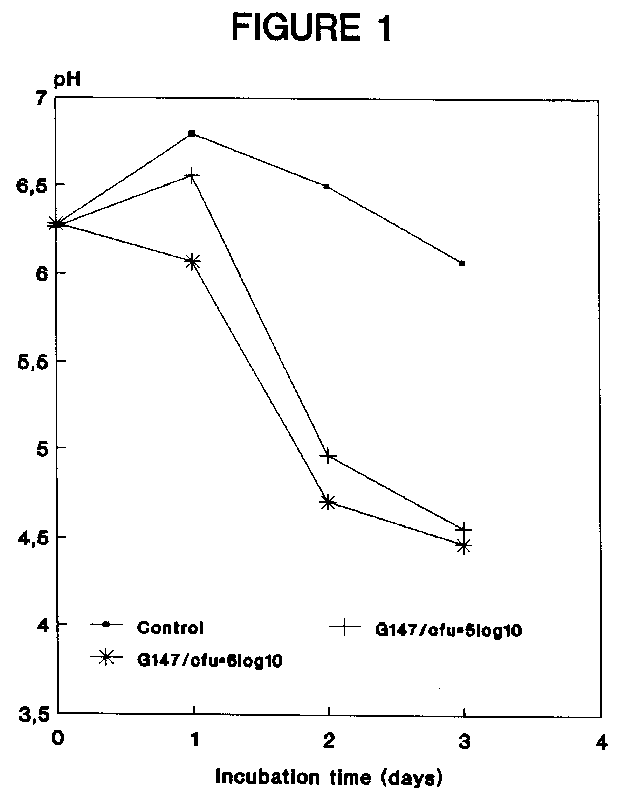

Results:
During the experiment the rate of acidification of the silage inoculated with strain G147 exceeded that of the control, and it was dependent on the inoculation level (Figure 1).

### Example 3

### Anti-clostridium activity in grass silage

Just like in Example 2 grass cut up into pieces of about 2 cm was used. Care was taken to use nitrate-free grass, in order to prevent clostridia-inhibition by nitrate-degradation.

In experiments 3.1, 3.2 and 3.3 the grass was inoculated with lactobacillus cells in an amount of 10⁵ and 10⁶ cfu/g of grass.

As a control demineralized water was used. For each treatment three 1l bottles with a septum (Experiments 3.1 and 3.2) or without a septum (Experiment 3.3) were filled with 400 g of grass/bottle.

The respective lactobacillus suspensions were sprayed onto the grass (25 ml of suspension/kg of grass). In experiments 3.2 and 3.3, apart from the Lactobacillus, soil extract was sprayed on to the grass (25 ml of extract/kg of grass) to increase the starting amount of clostridium spores. The soil extract was produced by mixing 1 kg of pasture soil and 780 ml of demineralized water

In experiment 3.4 the grass was inoculated with lactobacillus suspension at 10⁶ cfu/g. As a control demineralized water was used. For each treatment 3.5 kg of grass was filled into a 10 l laboratory silo. The respective lactobacillus strains were applied to the grass using a plant spray (25 ml/kg) and the grass was mixed using a concrete mixer. In order to increase the number of clostridium spores a soil suspension (1 kg of pasture soil + 750 ml of demineralized water) was applied (25 ml/kg of grass). The dry mass content of the grass was decreased by adding 100 ml demineralized water/kg.

During Experiments 3.1 and 3.2 the weight loss and the hydrogen concentrations of the silages were measured periodically.
For each treatment in Experiment 3.1 one bottle was removed after 21 days and its content was frozen.
For each treatment in Experiment 3.2 the contents of two bottles were removed after 26 days and frozen.
The numbers of clostridium spores and the contents of ethanol, volatile fatty acids, lactic acid and ammonia from these frozen samples were determined.

During Experiment 3.3 the contents of two bottles were removed after 6 weeks and the number of clostridium spores, and contents of ethanol, volatile fatty acids, lactic acid and ammonia were determined in these samples.

During Experiment 3.4 the hydrogen concentrations of each silo were determined periodically, and a sample of the silage was removed for determination of the pH, of the concentrations of ethanol, volatile fatty acids, lactic acid and ammonia, and of the numbers of lactic acid bacteria, yeasts, enterobacteria and clostridium spores.

Results Experiment 3.1. The results of this experiment after incubation of the grass during 21 days are summarized in Table 4. Herein RCM means the number of clostridium spores (log cfu/g) according to the RCM plate method; HAC means acetic acid content (mmol/kg), HB means butyric acid content (mmol/kg) and HL means lactic acid content (mmol/kg). Little differences were shown between the controls and the inoculated silages, all silages were well preserved (low pH) and contained only a low amount of clostridium spores (<10² cfu/g)

**Table 4**

| **Strain-cfu/g** | **RCM** | **HAC** | **HB** | **HL** | **pH** |
|---|---|---|---|---|---|
| Controle | <2.00 | 39.7 | 1.2 | 174 | 4.03 |
| G147-10⁵ | <2.00 | 34.0 | 0.0 | 122 | 3.88 |
| G147-10⁶ | <2.00 | 31.2 | 0.0 | 255 | 3.85 |
| F123-10⁵ | <2.00 | 35.1 | 0.0 | 245 | 3.88 |
| F123-10⁶ | <2.00 | 33.3 | 0.0 | 237 | 3.90 |

Results Experiment 3.2. After 6 days of incubation a minor increase of both hydrogen production and weight loss in the control silages as compared to the inoculated silages was found. The results summarized in Table 5 (data after 26 days of incubation) lend support to the hypothesis that this increase was due to clostridium activity.

Abbreviations in Table 5 have the same meaning as in Table 4; MPN means the number of clostridium spores (log cfu/g) according to the Most Probable Number (MPN) tube method.

**Table 5**

| **Strain-cfu/g** | **Spores** | | **HAC** | **HB** | **HL** | **pH** |
|---|---|---|---|---|---|---|
| | **RCM** | **MPH** | | | | |
| Control | 6.12 | 5.97 | 8.1 | 64.4 | 134 | 4.69 |
| | 6.08 | 5.88 | 5.6 | 62.0 | 131 | 4.70 |
| G147-10⁵ | 3.39 | 4.38 | 23.7 | 2.5 | 243 | 3.72 |
| | 4.61 | 4.97 | 21.7 | 3.4 | 248 | 3.72 |
| G147-10⁶ | 3.20 | 3.63 | 19.5 | 0.9 | 239 | 3.65 |
| | 2.48 | 3.85 | 20.3 | 0.0 | 241 | 3.65 |
| F123-10⁵ | 2.88 | 5.18 | 24.6 | 1.1 | 242 | 3.73 |
| | 3.00 | 4.97 | 23.2 | 1.2 | 248 | 3.71 |
| F123-10⁶ | 3.13 | 4.15 | 20.8 | 1.0 | 242 | 3.64 |
| | 4.76 | 4.88 | 24.0 | 0.0 | 234 | 3.67 |

Results Experiment 3.3. The observations made in Experiment 3.2 were confirmed in this experiment after 42 days of incubation: the control silages showed higher pH and butyric acid levels, more weight loss and much higher numbers of clostridium spores than the inoculated silages (Table 6; WL means weight loss (g/kg)).

**Table 6**

| **Strain-cfu/g** | **RCM** | **HAC** | **HB** | **HL** | **pH** | **WL** |
|---|---|---|---|---|---|---|
| Control | 5.35 | 33.5 | 86.0 | 175 | 5.36 | 25.3 |
| | 5.22 | 29.1 | 84.8 | 236 | 5.39 | 25.8 |
| G147-10⁵ | 2.30 | 21.0 | 1.6 | 219 | 3.70 | 3.47 |
| | <2.00 | 26.1 | 1.4 | 288 | 3.69 | 3.96 |
| G147-10⁶ | <2.00 | 21.8 | 3.2 | 286 | 3.68 | 4.01 |
| | 2.70 | 21.5 | 1.8 | 289 | 3.68 | 3.70 |
| F123-10⁵ | <2.00 | 24.1 | 1.6 | 284 | 3.68 | 4.68 |
| | 3.00 | 12.7 | 1.7 | 276 | 3.71 | 3.99 |
| F123-10⁶ | 2.90 | 16.2 | <1.0 | 280 | 3.67 | 5.23 |
| | 3.23 | 22.7 | <1.0 | 275 | 3.67 | 4.69 |

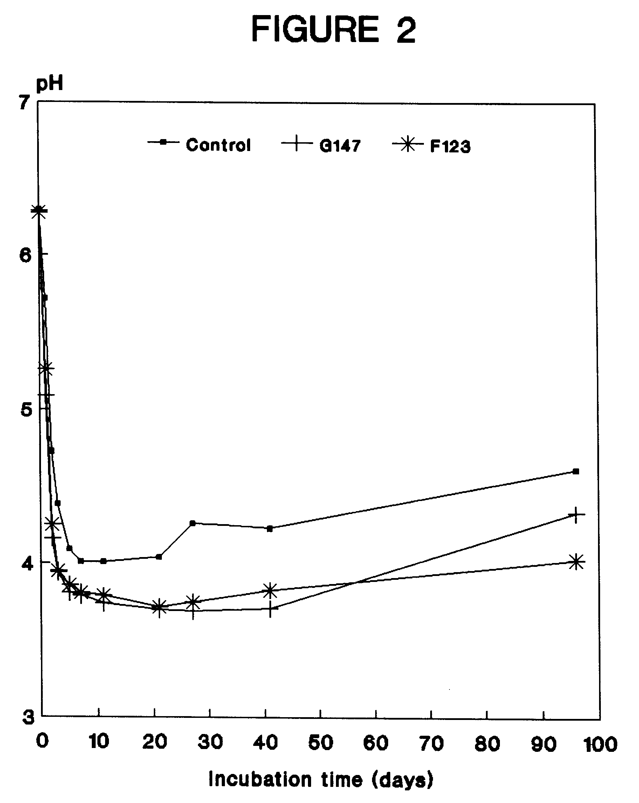

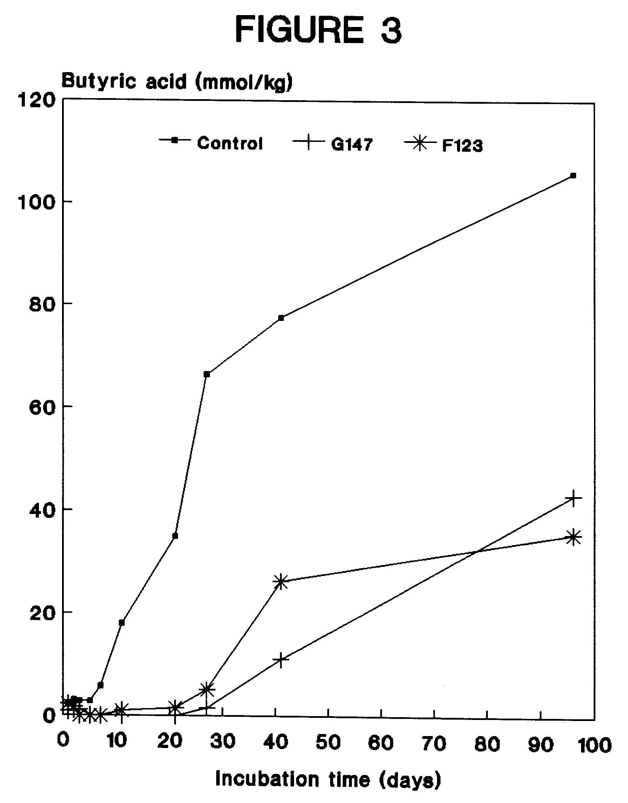

Results Experiment 3.4. It is shown in Figure 2 that the pH of the two inoculated silages dropped to about 3.8 within 5 days (pH 4.1 was found in the controls). The onset of a subsequent pH rise occurred much later in the inoculated silages (41 days) then in the control silages (21 days).

## Claims

1. Method for the production of silage by microbial conversion of silage crop during an aerobic phase and a subsequent anaerobic phase, characterized in that the anaerobic phase takes place in the presence of lactic acid bacteria which produce a Clostridium inhibiting factor.

2. Method according to claim 1, characterized in that the anaerobic phase takes place in the presence of lactic acid bacteria which produce a protenaceous Clostridium inhibiting factor.

3. Method according to claim 1, characterized in that the anaerobic phase takes place in the presence of Lactobacillus plantarum bacteria which produce a Clostridium inhibiting factor.

4. Method according to claim 3, characterized in that the anaerobic phase takes place in the presence of Lactobacillus plantarum bacteria which produce a protenaceous Clostridium inhibiting factor.

5. Use of lactic acid bacteria producing a Clostridium-inhibiting factor in the production of silage.

6. Use of lactic acid bacteria producing a proteinaceous Clostridium-inhibiting factor in the production of silage.

7. Use of Lactobacillus plantarum bacteria producing a Clostridium-inhibiting factor in the production of silage.

8. Use of Lactobacillus plantarum bacteria producing a proteinaceous Clostridium-inhibiting factor in the production of silage.

9. Lactobacillus plantarum bacteria which are able to produce a Clostridium-inhibiting factor.

10. Lactobacillus plantarum bacteria which are able to produce a proteinaceous Clostridium-inhibiting factor.

11. Silage inoculant containing Lactobacillus bacteria according to claim 9-10.
